# EUROPÄISCHE PATENTSCHRIFT

(11) **EP 2 964 092 B1**
(45) Veröffentlichungstag und Bekanntmachung des Hinweises auf die Patenterteilung: **11.09.2019**
(21) Anmeldenummer: 14713770.7
(22) Anmeldetag: 28.02.2014
(51) Int. Cl.: A61B 5/20

(54) **DRUCKMESSSYSTEM UND VERFAHREN ZUR AUTOMATISCHEN ENTLÜFTUNG UND NULLSTELLUNG**
PRESSURE-MEASURING SYSTEM AND METHOD FOR AUTOMATIC DEAERATION AND ZERO-SETTING
SYSTÈME DE MESURE DE PRESSION ET ET PROCÉDÉ AUTOMATIQUE DE DÉSAÉRATION DE MISE À ZÉRO

(30) Priorität: 04.03.2013 DE 102013102083
(43) Veröffentlichungstag der Anmeldung: 13.01.2016
(73) Patentinhaber: Medical Measurement Systems B.V., 7521 PV Enschede (NL)
(72) Erfinder: JENSEN, Michael Gondy, DK-4450 Jyderup (DK); WITTE, Jens, 81476 München (DE); VAN GORKOM, David, 83209 Prien am Chiemsee (DE); LARSEN, Kristine, 81476 München (DE)
(74) Vertreter: Wittmann, Ernst-Ulrich
(86) Internationale Anmeldenummer: PCT/EP2014/053968
(87) Internationale Veröffentlichungsnummer: WO 2014/135457

(56) Entgegenhaltungen:
- WO-A1-98/23320
- DE-A1- 10 032 616
- DE-A1- 19 532 332
- US-A- 4 621 647
- US-A- 6 056 697
- US-A1- 2004 006 321
- US-A1- 2007 038 143

## Beschreibung

Die vorliegende Erfindung betrifft ein Druckmesssystem, insbesondere für die medizinische Diagnose, ein Verfahren zur Entlüftung und Nullstellung des Druckmesssystems und die Verwendung des Systems im Bereich der Urodynamik und Gastroenterologie.

Im Stand der Technik ist die diagnostische Druckmessung insbesondere in der Urodynamik bekannt. Die Druckmessung mit wassergefüllten Einmalkathetern ist die in der Urodynamik am weitesten verbreitete Methode und wird von der ICS (International Continence Societe = Weltweiter Dachverband für die Urodynamik) wegen der Messqualität und Robustheit als "Golden Standard" empfohlen.

Die US 4,621,647 A offenbart eine Vorrichtung zur Messung des intrakraniellen Gehirnflüssigkeitsdrucks. Dabei wird ein Verfahren für das Leeren des Schlauchsystems der Vorrichtung und für die Nullstellung auf Atmosphärendruck offenbart.

Die US 2007 038 143 A1 offenbart eine Vorrichtung zur Druckmessung des intraabdominalen Drucks eines Patienten unter Verwendung eines urethralen Katheters. Ein Hauptteil der Fluide wird über eine Pumpe und ein Ventil geleitet. Ein zweiter Weg der Fluide über einen Durchfluss-Minderer ist angelegt, um den Drucksensor von Drücken zu entlasten, die vom Infusionsbeutel her rühren.

Die DE 100 32 616 A1 offenbart eine Messkammer mit einer Membran und einer darunter liegenden Transducermembran, die mit einem einem Sensor (Transducer) zur Umwandlung von Drücken und Druckänderungen in elektrische Signale verbunden ist.

Die US 2004 006 321 A1 betrifft ein System zur Urinhandhabung. Dieses weist insbesondere ein dochtartiges Element auf, das in den Ableitungsschlauch gelegt wird und das auch bei einem Knick im Schlauch den kontinuierlichen Abfluss gewährleistet.

Die US 6,056,697 A offenbart ein System und ein Verfahren zur Kalibrierung von Drucksensoren, insbesondere für Blutdruck, an der Spitze von Kathetern. Dazu werden die Katheter in ein Gefäß mit einer salzhaltigen Lösung in einer definierten Tiefe eingeführt. Die gemessenen hydrostatischen Drücke dienen als Basis zur Kalibrierung des Systems.

Die DE 195 32 332 A1 offenbart ein System, das Veränderungen des hydrostatischen Druck, der sich bei der Veränderung der Liegehöhe eines Patienten ergibt, durch einen parallel angeordneten, wassergefüllten Schlauch kompensiert.

Bei der Druckmessung mit wassergefüllten Kathetern wird der Druck im Körper über eine Wassersäule, welche durch die Lumen der Katheter und die Druckübertragungsschläuche geführt wird, auf externe Drucksensoren geleitet. Dort misst man dann den Druck im Körper inkl. einen hydrostatischen Druck, der sich aus der Höhendifferenz der Messwertgeber zum Messort ergibt. Dieser Druckoffset wird entweder durch eine definierte Positionierung der Sensoren (Os Pubis-Höhe In der Urodynamik) oder durch elektronisches Nullsetzen kompensiert.

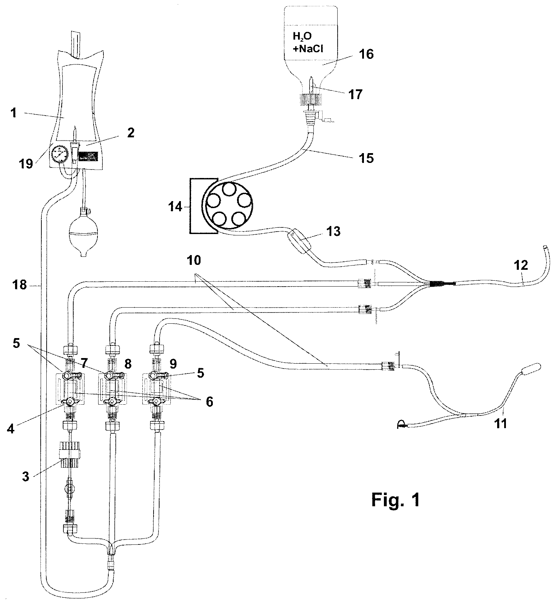

Heute bedarf eine urodynamische Untersuchung einen erheblichen Aufwand in der Vorbereitung und es wird eine erhebliche Anzahl an sterilen Einmalartikel benötigt. Ein Pumpenschlauch, ein Perfusionsschlauch, drei Druckmesswertgeber mit jeweils einem Drei- und einem Zweiwegehahn, drei Druckübertragungssohläuche, ein Transurethralkatheter mit einem Füll- sowie zwei Messvolumen und einem Rektalkatheter mit Ballon, welche alle am Messplatz aufgebaut und vorbereitet werden müssen (Figur 1).

Die Stärken dieser im Stand der Technik bekannten Methode ist u.a. die absolut überlegene Messqualität durch überragende Impulsantwortzeiten, hohe Nullpunktstabilität, niedrige Temperaturdrift und niedrige Hysterese. Ferner ist auch die systembedingte, automatische Kompensation des Druckoffset bei Verschiebung des Messvolumens in der Blase bedeutungsvoll. Darüber hinaus kann die Änderung des hydrostatischen Drucks in der Blase inhärent durch die daran gekoppelte Höhe der Wassersäule der Druckübertragungsschläuche zu den Druckmesswertgebern ausgeglichen werden.

Nachteilig ist jedoch die sehr komplizierte, fehlerträchtige und zeitraubende Handhabung. Jeder der verschiedenen Einzelschritte muss in Anwesenheit eines in den meisten Fällen unruhigen bis nervösen Patienten manuell ausgeführt werden, was auch bei erfahrenen Anwendern häufig zu Fehlern führt und es ist eine große Anzahl von einzelnen Einmalartikeln notwendig, welche häufig zu logistischen Problemen führen.

Ausgehend von diesem Stand der Technik ist es nun Aufgabe der vorliegenden Erfindung ein Druckmesssystem bereit zu stellen, mit welchem die im Stand der Technik bekannten Nachteile wenigstens teilweise überwunden bzw. verbessert werden.

Gelöst wird diese Aufgabe durch ein erfindungsgemäßes Druckmesssystem gemäß Anspruch 1, ein Verfahren zur automatischen Entlüftung und Nullstellung eines entsprechenden Druckmesssystems gemäß Anspruch 20. Bevorzugte Ausgestaltungsformen des Systems und des Verfahrens sind Gegenstand der jeweiligen Unteransprüche. Die Erfindung umfasst auch die Verwendung des Druckmesssystems im Bereich der Urodynamik und Gastroenterologie, insbesondere zur Blasendruckmessung.

Die vorliegende Erfindung umfasst ein Druckmesssystem, mit wenigstens einen, mit einem fluidfüllbaren Messlumen, wenigstens ein Schlauchsystem zur Verbindung eines Fluidreservoirs mit dem wenigstens einen Messlumen und einer Anordnung oder Vorrichtung zur Beförderung des Fluids durch das wenigstens eine Schlauchsystem. Die Erfindung ist dadurch gekennzeichnet, dass das Schlauchsystem wenigstens eine erste Vorrichtung zur Druckmessung in einem bzw. dem Fluid umfasst und diese Vorrichtung aus einem Kopplungselement, wenigstens einen Druckwandler und wenigstens einer, mit einem oder der fluidbefüllbaren Messkammer aufweist. Das Kopplungselement verbindet vorzugsweise die Messkammer über eine Membran mechanisch mit der Messfläche des Druckwandlers, wobei die Messkammer wenigstens zwei Anschlussstellen für das Fluid, bzw. den Fluidstrom aufweist. Darüberhinaus weist das Schlauchsystem zwischen der ersten Vorrichtung zur Druckmessung und der Zuführung des Fluids eine zweite Vorrichtung zur Regulation eines Volumenstroms auf, welcher neben einem fluid-durchströmbaren Schlauchabschnitt wenigstens eine Schlauchklemme umfasst. Darüberhinaus ist die zweite Vorrichtung derart gestaltet, dass diese in Regulationsstellungen offen, geschlossen und perfundiert einstellbar ist, wobei in der geschlossenen Stellung das Lumen des Schlauchabschnittes vollständig geschlossen und in der perfundierten Stellung das Lumen des Schlauchabschnittes durch einen Abstandhalter im Inneren des Schlauchabschnittes, welcher wenigstens im Bereich der Klemmstelle der Schlauchklemme hineinreicht, teilweise, insbesondere geringfügig offen ist.

Als Fluid gemäß der vorliegenden Erfindung werden fließfähige Systeme insbesondere Flüssigkeiten betrachtet, die beispielsweise in der Medizin oder Medizintechnik verwendet werden. Solche sind beispielsweise Infusionslösungen wie Natriumchlorid Lösungen, Wasser, wässerige Lösungen, Injektionslösungen, Infusionslösungen, Nährlösungen, Elektrolytlösungen, Blut, Plasma, Gas, Luft, Kombinationen hieraus und dergleichen.

Diese Fluide werden gemäß einer weiteren besonders bevorzugten Ausführungsform in Fluidreservoiren bevorratet, die auch wiederrum im Stand der Technik wie beispielsweise in der Form von Infusionsflaschen oder Infusionsbeutel bekannt sind.

Als Messlumen wird gemäß der vorliegenden Erfindung der flüssigkeitsfüllbare Gesamtraum eines Schlauchsystems verstanden, welcher gemäß einer besonders bevorzugten Ausführungsform vom Fluidreservoir bis beispielsweise zum Öffnungsabschnitt eines Anwendungssystems wie z.B. eines Katheters verstanden wird. In Abgrenzung zum Messlumen wird als Messkammer ein Behälter, beziehungsweise ein Raumvolumen verstanden, welches im unmittelbaren Bereich zum Druckwandler, d.h. zur Vorrichtung zur Bestimmung des Druckes angeordnet ist und in welchem vorzugsweise neben den Strömungsverhältnissen auch das hierin begrenzte Volumen bestimmt ist.

Als Kopplungselement gemäß der vorliegenden Erfindung wird ein oder eine Vielzahl von Abschnitten der Vorrichtung zur Druckmessung verstanden, mit welchen vorzugsweise die mit dem Fluid befüllbare Messkammer und deren durch eine Membran bestimmte Messfläche mit dem Druckwandler bzw. Drucksensor derart verbunden wird, dass der Druck innerhalb der Messkammer bestimmbar ist. Solche Kopplungselemente können beispielsweise Rastelemente oder lösbare Steckverbindungen wie beispielsweise Bajonett oder ähnliche Verbindungen, aber auch mechanisch arretierbare Verbindungssysteme sein.

Gemäß der vorliegenden Erfindung kann wenigstens die zweite Vorrichtung zur Regulation des Volumenstroms in drei Regulationsstellungen eingestellt werden, welche die Positionen offen, geschlossen und perfundiert umfasst. Gemäß der vorliegenden Erfindung wird unter perfundiert eine Einstellung verstanden, bei welcher unter vorgegebenen Bedingungen der Volumenstrom, welcher durch die Vorrichtung zur Regulierung des Fluidstroms bestimmbar ist zwischen 0,1 ml pro Minute und 8 ml pro Minute vorzugsweise zwischen 0,5 ml pro Minute und 5 ml pro Minute begrenzt wird. Solch eine Einstellung ist dann von Nöten, wenn insbesondere sehr geringe Volumenströme notwendig sind um die entsprechende Druckmessung in einem dynamischen System vornehmen zu können. Solche Volumenströme werden gemäß der vorliegenden Erfindung als geringfügig betrachtet, da sie im Vergleich zum normalen bzw. maximalen Volumenstrom als relativ klein zu bezeichnen sind.

Das Kopplungselement ist gemäß einer weiteren, besonders bevorzugten Ausführungsform eine Klemmkante, welche vorzugsweise wenigstens eine drehbar gelagerte, kraft- und/oder formschlüssig mit der Kopplungseinheit verbindbare Andruckwalze aufweist und die Messkammer darüberhinaus zwei gegenüberliegende äußere Stege umfasst, die in eine Klemmkante oder in die Andruckwalze eingreifen. Hierbei umfasst vorzugsweise die Andruckwalze einen im Wesentlichen sich axial erstreckenden Schlitz, in welchen einer der Stege der Messkammer einführbar ist und über diese kraft- und/oder formschlüssig verbindbar ist. Gemäß einer weiteren besonders bevorzugten Ausführungsform ist die Andruckwalze manuell oder mittels eines Motors, insbesondere eines Schrittmotors oder eines Serienmotors bewegbar und wird entweder mechanisch oder mittels dem Motor in einer vorgegebenen Position gehalten beziehungsweise arretiert. Diese Positionen sind wenigstens die Position offen, d.h. eine Position in welcher der Steg der Messkammer einführbar ist und eine Position geschlossen, in welcher dieser Steg arretiert ist.

Gemäß der vorliegenden Erfindung umfasst die zweite Vorrichtung zur Regulierung des Volumenstroms wenigstens eine Schlauchklemme, die vorzugsweise in einem Grundgehäuse mit einem Aufnahmebereich für den entsprechenden Schlauchabschnitt angeordnet ist. Hierbei umfasst die Schlauchklemme vorzugsweise auch ein Klemmelement, welches in Wechselwirkung mit wenigstens einem, vorzugsweise einer Vielzahl von Abschnitten des Aufnahmebereichs in dem Grundgehäuse als Andruckstellen stehen können und somit wenigstens zwei Regulationsstellungen bereitstellen, in welchen das Lumen des Schlauchabschnittes geöffnet, geschlossen und/oder perfundiert d.h. geringfügig offen ist.

Gemäß einer besonders bevorzugten Ausführungsform der vorliegenden Erfindung ist das Druckmesssystem dadurch gekennzeichnet, dass die Regulationsstellungen durch wenigstens eine Schlauchklemme bewirkt werden, wobei das Klemmelement drehbeweglich angeordnet ist und sich radial um den Drehpunkt erstreckt. Insbesondere der zum Drehpunkt nächstgelegene Abschnitt des Klemmelements wirkt hierbei mit wenigstens einer Andruckstelle der Gehäuseaufnahme beziehungsweise des Gehäuseaufnahmebereiches zusammen.

In einer weiteren, besonders bevorzugten Ausführungsform umfasst dabei die Schlauchklemme ein Grundgehäuse mit einem Aufnahmebereich für einen Schlauchabschnitt und ein, zwischen zwei bzw. drei Regulationsstellungen bewegbares Klemmelement. Dabei wirkt das Klemmelement in Wechselwirkung mit Abschnitten des Aufnahmebereiches als Andruckstellen wenigstens in einer Regulationsstellung auf den Schlauchabschnitt und dessen Lumen ein. Bevorzugt ist gemäß einer weiteren Ausführungsform der Erfindung im Bereich wenigstens einer Regulationsstellung im Lumen des entsprechenden Schlauchabschnittes ein Abstandshalter angeordnet.

In einer alternativen, aber auch besonders bevorzugten Ausführungsform wird das Klemmelement mittels eines Motors, insbesondere eines Schrittmotors oder eines Servomotors zwischen den Regulationstellen bewegt und vorzugsweise in diesen entsprechend gehalten bzw. arretiert. Hierbei können für die einzelnen Positionen Arretiermechanismen vorgesehen sein oder der entsprechende Schrittmotor oder Servomotor bietet an den entsprechenden Positionen Arretierungen. Alternativ hierzu kann das Klemmelement auch händisch in entsprechende Positionen bewegt werden und ggf. mechanisch fixiert werden.

Gemäß einer weiteren besonders bevorzugten Ausführungsform der vorliegenden Erfindung ist der Schlauchabschnitt aus einem flexiblen Material hergestellt, wobei dieses insbesondere ein flexibler Kunststoff ist, der vorzugsweise abschnittsweise quetschbar ist. Solche Schlauchabschnitte oder Schläuche sind im Stand der Technik bekannt. Darüberhinaus weist ein entsprechender Schlauchabschnitt ein Lumen auf, d.h. er hat einen lichten Durchmesser, welcher zwischen 0,2 mm und 10 mm vorzugsweise zwischen 0,5 mm und 5 mm und insbesondere bei ca. 3 mm liegt.

Darüberhinaus können wenigstens in einem Schlauchabschnitt als integraler Bestandteil ein Abstandshalter vorgesehen, wobei gemäß einer weiteren besonders bevorzugten Ausführungsform dieser Abstandshalter das Lumen des Schlauches ungleichmäßig reduziert und/oder der Abstandhalter durch ein längliches, insbesondere ein faden- oder stabförmiges, das Lumen des Schlauchs ungleichmäßig reduzierendes Element gebildet wird. Solche Abstandshalter können in den Mantel des Schlauches integriert oder vorzugsweise mit dem Schlauchabschnitt kraft-, form- und/oder stoffschlüssig verbunden sein. Hierbei reduzieren diese Abstandshalter gemäß einer weiteren, besonders bevorzugten Ausführungsform das Lumen des entsprechenden Schlauches beziehungsweise Schlauchabschnittes zwischen 0,01 % bis 5 %, bevorzugt zwischen 0,1 % und 1 % und insbesondere in einem Bereich von ca. 0,5 %.

Ziel dieses Abstandhalters ist es gemäß der vorliegenden Erfindung in der entsprechenden Regulationsstellung der Vorrichtung zur Regulierung des Volumenstroms den Schlauchabschnitt in einer so-genannten "perfundierten" Weise offen zu halten, so dass nur geringfügige Volumenströme des Fluids hindurchfließen können.

Gemäß einer weiteren, besonders bevorzugten Ausführungsform des erfindungsgemäßen Drucksystems sind die Anschlussstellen zur Messkammer in einem Winkel α angeordnet, welcher zwischen 180° und 60°, bevorzugt zwischen 180° und 120° und insbesondere in einem Winkel zwischen 144° bis 115° besonders bevorzugt von ca. 150° zueinander angeordnet sind. Darüberhinaus und/oder entsprechend zu diesem Winkel α sind die Anschlussstellen in einem Winkel β zur Grundfläche der Messkammer angeordnet, welcher zwischen 0° und 60°, vorzugsweise zwischen 12° und 18° und insbesondere von kleiner 60° und insbesondere von ca. 15° liegt.

Die Messkammer kann ferner gemäß einer weiteren, besonders bevorzugten Ausführungsform derart ausgestaltet sein, dass diese eine kreisrunde Grundfläche aufweist, in welcher gemäß einer weiteren besonders bevorzugten Ausführungsform die Membran, welche entsprechend unmittelbar bzw. mittelbar mit dem Druckwandler gekoppelt ist, angeordnet wird. Diese Grundfläche kann ggf. auch offen sein, wobei vorzugsweise die Grundfläche durch die zuvor genannte Membran abgedeckt und die Messkammer mit dieser Membran fluiddicht verbunden ist. Solche Membranen werden vorzugsweise aus einem Material hergestellt, welches aus einer Gruppe ausgebildet ist, die, Silikon, Latex, Kautschuk, Kombinationen hiervon oder dergleichen umfasst.

Gemäß einer weiteren besonders bevorzugten Ausführungsform der vorliegenden Erfindung ist der Druckwandler, welcher über die Membran den Druck in der Messkammer bestimmt ein sogenannter mechano-elektrischer Druckwandler, dessen Messfläche vorzugsweise formschlüssig mit der zuvor genannten Membran bedeckt wird und/oder der Messbereich des Druckwandlers zentrisch im Bezug auf die Grundfläche der Messkammer angeordnet ist. Die Messkammer hat ferner gemäß einer weiteren, besonders bevorzugten Ausführungsform der vorliegenden Erfindung einen Innenraum, der im Wesentlichen kuppelförmig gestaltet ist und dessen Innenraum gemäß einer weiteren besonders bevorzugten Ausführungsform im zentralen Bereich abgeflacht ist.

In einer weiteren besonders bevorzugten Ausführungsform der vorliegenden Erfindung wird wenigstens die Schlauchklemme und/oder das Kopplungselement und/oder die Messkammer wenigstens Abschnittsweise aus einem Material hergestellt, das aus einer Gruppe ausgewählt ist, welche vorzugsweise duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan ungesättigtes Polyesterharz, Acrylnitril-Butadien-Styrol, Acrylester-Styrol-Acrylnitril, Metalle wie zum Beispiel Edelstahl, Aluminium, Kombinationen hiervon und dergleichen umfasst.

Darüberhinaus ist die Vorrichtung zur Beförderung des Fluids gemäß der vorliegenden Erfindung vorzugsweise eine Peristaltikpumpe bzw. eine Verdrängungspumpe und insbesondere eine Schlauchpumpe bzw. eine Rollpumpe. Darüberhinaus können auch entsprechende Infusionspumpen verwendet werden. Alternativ zum Einsatz einer entsprechenden mechanischen oder hydraulischen Pumpe liegt es auch im Sinne der vorliegenden Erfindung, dass einfach durch die Anordnung des entsprechenden Fluidreservoir eine so-genannte Schwerkraftpumpe bereitgestellt wird.

Neben der zuvor beschriebenen Vorrichtung der vorliegenden Erfindung wird die Aufgabe der Erfindung auch durch ein Verfahren zur automatischen Entlüftung und Nullstellung eines Drucksystems nach Positionierung wenigstens eines Messlumens gelöst. Hierbei umfasst das Verfahren neben dem Schritt der Messung des Hohedrucks am Druckwandler bei luftgefüllten Schlauchsystemen und Messlumen das Befüllen des Schlauchsystems und des Messlumens mit einem Fluid aus dem Fluidreservoir und die anschließende Korrektur des durch den Höhenunterschied zwischen dem Sensor bzw. der Messkammer und dem Messlumen bzw. dem Messort wirkenden hydraulischen Drucks durch Abgleich mit dem vorher bestimmten Ruhedrucks. Solch ein Verfahren ist in Kombination mit der zuvor beschriebenen Vorrichtung Gegenstand der vorliegenden Erfindung. Darüberhinaus kann in einer weiteren besonders bevorzugten Ausführungsform der vorliegenden Erfindung der gemessene Ruhedruck an ein Steuergerät übertragen werden und aus den Messwerten, d.h. einer Vielzahl an Messwerten ein mittlerer Ruhedruck bestimmt werden.

Gemäß einer weiteren, besonders bevorzugten Ausführungsform umfasst das Verfahren die Korrektur des durch den Höhenunterschied zwischen der Messkammer und dem Sensor wirkenden hydrostatischen Drucks durch Abgleich mit dem mittleren Ruhedruck. Die zum Befüllen verwendete Menge an Fluid entspricht gemäß einer weiteren besonders bevorzugten Ausführungsform der vorliegenden Erfindung im Wesentlichen dem Volumen des Schlauchsystems und des Messlumens und/oder des Infusionsvolumens.

Gemäß einer weiteren, besonders bevorzugten Ausführungsform wird mit dem erfindungsgemäßen Verfahren der Befüllvorgang automatisch beendet, wenn ein dem Volumen des Schlauchsystems und des Messvolumens und/oder des Infusionsvolumens entsprechende Menge aus dem Fluidreservoir in das Schlauchsystem und das Messlumen gepumpt, beziehungsweise zugeführt wurde. Hierbei kann gemäß einer weiteren besonders bevorzugten Ausführungsform die Menge des in das Schlauchsystem und dass Messlumen oder Infusionsvolumen gepumpte Fluid über Umdrehungen des Rollrades der Pumpe oder über einen Volumentransducer bestimmt werden. Als weiteren, besonders bevorzugten Schritt des erfindungsgemäßen Verfahrens wird vor dem Befüllen des Schlauchsystems und des Messlumens die Schlauchklemme als Bestandteil der Vorrichtung zur Regulation des Fluidstroms automatisch in die geöffnete Regelungsstellung gebracht und nach dem Befüllen wieder in die geschlossene Regelungsstellung gestellt. Darüberhinaus liegt es im Sinne der vorliegenden Erfindung und dem beanspruchten Verfahren, dass die einzelnen Verfahrensschritte bei Verwendung mehrerer Drucksysteme wiederholt werden und vorzugsweise die einzelnen Verfahrensschritte vollautomatisch nach dem Start des Verfahrens durchgeführt werden. Wie bereits zuvor ausgeführt liegt es im Sinne des vorliegenden Erfindung, dass das zuvor beschriebene Druckmesssystem zur Durchführung des entsprechenden erfindungsgemäßen Verfahrens verwendet wird.

Die Erfindung umfasst neben der Vorrichtung zur Druckmessung auch die Verwendung des Druckmesssystems wie zuvor dargestellt im Bereich der Urodynamik und Gastroenterologie, insbesondere zur Blasen-, Rektal- und Urethraldruckmessung.

Nachfolgend wird die Erfindung anhand eines bevorzugten Ausführungsbeispiels beschrieben, wobei darauf hingewiesen wird, dass die Erfindung nicht auf die hier dargestellte Ausführung beschränkt, sondern vielmehr auch entsprechende Abwandlungen im Sinne der vorliegenden Erfindung sind.

Dabei zeigen:
- Figur 1: den Aufbau eines Messsystem gemäß dem Stand der Technik;
- Figur 2: den Aufbau eines Druckmesssystems gemäß der vorliegenden Erfindung;
- Figuren 3a - 3h: Detailansichten der Vorrichtung zur Regulation des Volumenstroms;
- Figur 4: eine Detailansicht des Pumpenschlauchs für das erfindungsgemäße Druckmesssystems;
- Figur 5: eine Explosionsansicht für die Zusammenstellung der Druckdomkassette mit den Druckmesssensoren;
- Figuren 6a bis 6d: Detaildarstellungen der Domkassette und deren Verriegelungsanordnung auf den Druckmesssensoren;
- Figur 7: Messprotokoll einer automatischen Entlüftung und Nullstellung des erfindungsgemäßen Druckmesssystems.

In Figur 1 ist schematisch der Aufbau einer Vorrichtung zur Druckmessung mit wassergefüllten Einmalkathetern dargestellt. Dabei ist neben dem Beutel mit einer Infusionslösung 1 und einer Druckmanschette 2, ein Druckminderer 3 und Zweiwegehähne 4 vor den Drucksensoren 6 dargestellt. Im Anschluss hieran sind Dreiwegehähne 5 angeordnet und die entsprechenden Leitungen werden als Pᵤᵣₐ 7, Pᵥₑₛ 8 und P_{abd} 9 bezeichnet. Über die Druckübertragungsschläuche 10 wird die Infusionslösung aus dem Beutel 1 zu dem Rektalkatheter 11 und den UDP-Katheter 12 geleitet. Darüberhinaus wird zum UDP-Katheter 12 aus der Flasche 16 mit z.B. einer Kochsalzlösung über die Rollenpumpe 14 und die Tropfkammer 13 die Kochsalzlösung aus der Flasche 16 definiert zugeführt.

Hiermit wird für die Vorbereitungs- und Messprozedur wie folgt verfahren:
a) Der Pumpenschlauch 15 wird in die Pumpe 14 eingelegt und der Dorn 12 des Schlauchs in den Flaschenstopfen eingesetzt. Die Pumpe 14 wird eingeschaltet bis die Tropfenkammer 13 der Flasche 16 halb gefüllt und der Schlauch komplett luftblasenfrei mit Kochsalzlösung gefüllt ist.
b) Die drei Druckmesswertgeber 6 werden in den Halterungen platziert und mit dem Perfusionsschlauch 18 verbunden - zwischen den Perfusionsschlauch und einen Druckmesswertgeber wird ein Durchflußminderer 3 eingesetzt. Der Dorn 19 des Perfusionsschlauchs 18 wird in die Wasserbeutel 1 eingesteckt.
c) Die Druckübertragungsschläuche 10 werden mit den Druckmesswertgebern 6 verbunden.
d) Alle Zwei- 4 und Dreiwegehähne 5 werden geschlossen und die Druckmanschette 2 wird aufgepumpt um die Wasserbeutel 1 unter Druck zu setzen.
e) Zum Entlüften der Druckübertragungslinien werden die Zwei- 4 und Dreiwegehähne 5 der Drucktransducer auf "offen" gestellt und blasenfrei bis an die Spitze mit Wasser gefüllt und anschließend der Zweiwegehahn 2 wieder geschlossen. Diese Prozedur muss für alle drei Druckübertragungslinen einzeln durchgeführt werden.
f) Die Dreiwegehähne 5 werden jetzt einzeln in "90 grad"- Position gedreht um die Druckkanäle per Tastendruck elektronisch auf Atmosphäre abzugleichen. Die Dreiwegehähne 5 werden anschließend wieder auf die Position "offen " gedreht.
g) Die beiden Katheter 11, 12 werden in Urethra und Rektum des Patienten platziert und an die Druckübertragungslinien sowie den Pumpenschlauch 10 angeschlossen.
h) Die Dreiwegehähne 5 müssen jetzt noch mal einzeln geöffnet um die beiden Lumen des Transurethralkathers 12 bis an die Spitze zu entlüften und den Ballon des Rektalkatheters 11 zu füllen.
i) Die gemessene Drücke werden überprüft und wenn notwendig per Software auf Null gestellt. Das System ist jetzt messbereit.

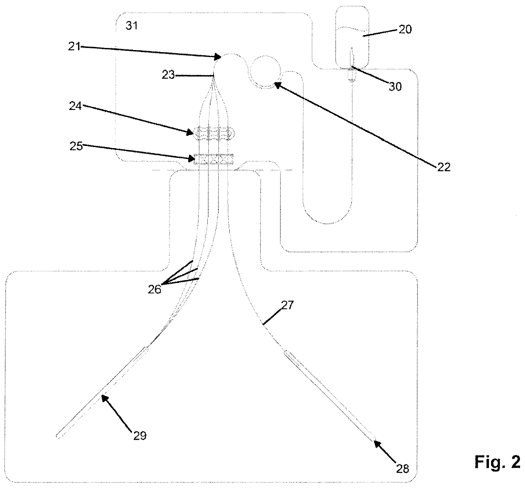

In Figur 2 ist der Aufbau eines erfindungsgemäßen Druckmesssystems dargestellt.

Dieses zeigt mit dem Bezugszeichen 21 den Pumpenschlauch des zu der Druckdomkassette 25 mit der Luer-Locks für den Anschluss von Kathetern 28 und 29 führt. Ferner ist der Einstichdorn 30 für Standard-Infusionbeutel 20 dargestellt. Mit dem Bezugszeichen 29 ist der Transurethraler und mit dem Bezugszeichen 28 der Rektalkatheter (ggf. auch beliebig viele Katheter oder Messvolumen), bezeichnet, welche mit dem Fluid über die Leitungen 26 (drei Stufen) und 27 versorgt werden. Hieran anschließend ist der Verriegelungsmechanismus der Domkassette 25 angeordnet. Im Bereich des Bezugszeichens 24 sind vorliegend vier (ggf. beliebig viele) Schlauchklemmen mit den Zuständen "offen", "geschlossen" und "perfundiert" angeordnet. Mit dem Bezugszeichen 22 ist eine Rollpumpe zur Beförderung des Mediums durch das Schlauchsystem dargestellt. Das System umfasst ferner ein Steuerungssystem 31 mit vollautomatischen Algorithmus zur Entlüftung des Schlauchsystems mit Kathetern, Auffindung von Ruhedrücken und Nullstellung des Messsystems unter Ausnutzung der Funktionalität der Einzelkomponenten.

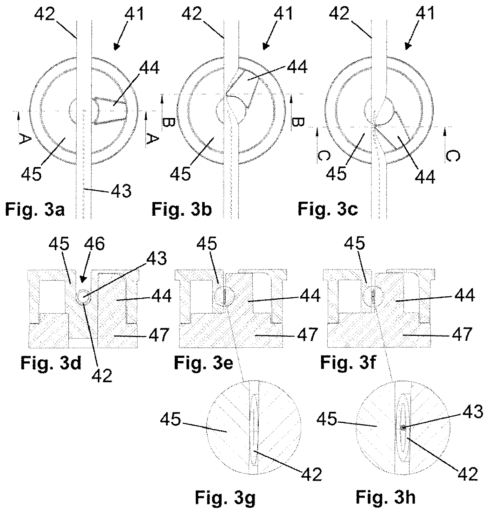

In den Figuren 3a - 3h sind unterschiedliche Stellungen (Offen, Geschlossen, Perfundiert) der Vorrichtung 41 zur Regulierung des Volumenstroms d.h. der Schlauchklemme gezeigt.

Hierbei ist in der oberen Reihe 3a bis 3c die Draufsicht auf eine entsprechende Vorrichtung 41 gezeigt, bei welcher der Schlauch 42 eingelegt ist. Im Schlauch ist in der unteren Hälfte ein Faden 43 im Sinne eines Abstandshalters zu erkennen. Ferner ist das Klemmelement 44 und die hiermit zusammenwirkbare Gegenwand 45 der Vorrichtung 41 dargestellt. In Figur 3a ist die offene Stellung, in der Figur 3b die vollständig geschlossene Stellung und in der Figur 3c die sogenannte perfundierte, d.h. die geringfügig geöffnete Stellung wiedergegeben.

In den Figuren 3d bis 3f sind Schnittdarstellungen aus den Figuren 3a bis 3c entlang der jeweiligen Schnittlinien A-A, B-B und C-C wiedergegeben. Auch hier ist der eingelegte Schlauch 42 mit dem Abstandshalter 43 zu erkennen, der in der Aufnahme 46 der Vorrichtung 41 eingelegt ist. Mit dem Bezugszeichen 44 ist das Klemmelement bezeichnet, welches auf einer drehbar gelagerten Führung 47 angeordnet ist. Mit dem Wandabschnitt 45 kann dieses in den entsprechenden Positionen (siehe 3c und 3f) zusammenwirken, wobei entsprechend der hier gezeigten Darstellung in der Position nach Figur 3b und 3e der Schlauch vollständig geschlossen ist und in der Position nach den Figuren 3c und 3f der Schlauch geringfügig geöffnet ist, obwohl das Klemmelement 44 mit der Gegenwand 45 zusammenwirkt. Das Abstandselement 43 bewirkt aber einen geringfügigen Fluidstrom trotz der bestehenden Quetschung des Schlauchs. In den Figuren 3g und 3h sind Detaildarstellungen der beiden Positionen 3e und 3h wiedergegeben, um den Unterschied zwischen geschlossen und perfundiert zu veranschaulichen. Deutlich ist die geringfügige Öffnung des Schlauches in der Fig. 3h zu erkennen.

Solch eine Vorrichtung 41 kann auch als Schlauchklemme bezeichnet werden. Die Schlauchklemmen ersetzen die in der konventionellen Druckmesstechnik verwendeten Zwei- und Dreiwegehähne. Zusätzlich erfüllen sie auch die Funktion des Perfusionsreglers für Druckmesskanäle, die perfundiert werden müssen (Urethradruck in der Urodynamik). Die Klemmen können durch einfaches Drehen der Achse mit einem beliebigen Aktuator in die Positionen "offen", "geschlossen" und "perfundiert" gebracht werden.

Position "offen": In dieser Position kann das Füllmedium die Schlauchklemmen ungehindert passieren. Sie ist die Start- und Endposition für alle Kanäle in der sich das Schlauchsystem einfach einlegen bzw. entnehmen lässt, wird zusätzlich für die Entlüftung der Messkanäle verwendet und stellt den normalen Arbeitszustand für den Infusionskanal dar.

Position "geschlossen": In dieser Position wird der Schlauch vollständig abgeklemmt und ist auch bei Druckdifferenzen von bis zu 2 bar unpassierbar für das Fluid. Sie ist der Arbeitszustand für die bereits entlüfteten Messkanäle und der vorübergehende Initialzustand für den Infusionskanal, wenn die Messkanäle entlüftet oder perfundiert werden.

Position "perfundiert": In dieser Position können, weitgehend unabhängig vom Vordruck, nur sehr geringe Mengen des Füllmediums (max. 8 ml/min) die Klemmstelle passieren. Das wird dadurch erreicht, indem der Schlauch zwar ähnlich wie in der Position "geschlossen" mit voller Kraft abgeklemmt wird, jedoch die Wände der Schläuche durch einen Abstandshalter wie zum Beispiel einen Faden immer eine kleine Öffnung freigehalten wird, die eine kapillaren Reduzierung des Füllmedienstroms bewirkt. Diese Position wird für Kanäle verwendet, die Perfusion erfordern (Urethadruck).

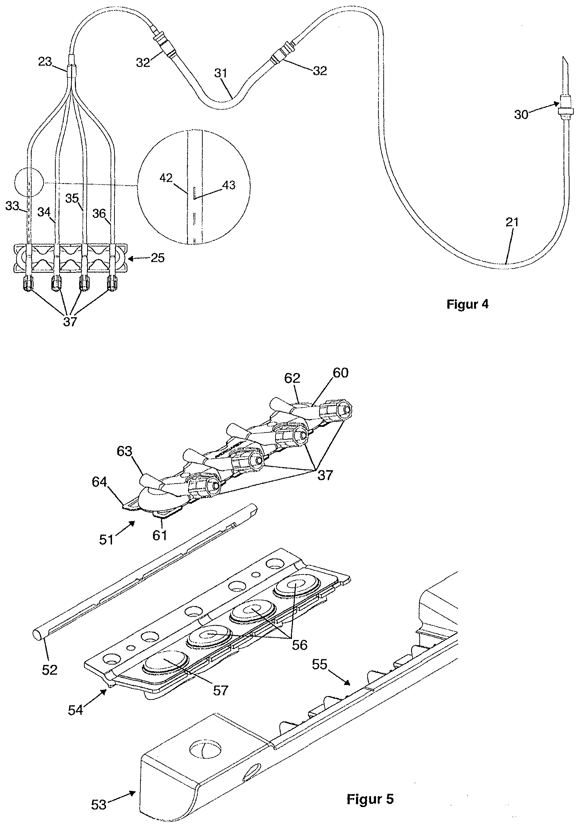

Im Pumpenschlauch - wie in Figur 4 gezeigt - sind der Infusionsschlauch 21, die Entlüftungsschläuche 34 bis 36 für die drei - ggf. beliebig viele - Druckkanäle und die Domkassette 25 integriert. Zudem besitzt der Pumpen- bzw. Infusionsschlauch 21 einen Anstichdorn 30 für einen Infusionsbeutel und Luer-Locks 37 für den Anschluss der Katheter. Bei der Verwendung des Systems mit einer Rollenpumpe weist das Schlauchsystem in diesem Bereich einen geeigneten kompressiblem Schlauchabschnitt 31 auf, der durch entsprechende Verbinder 32 in das Schlauchsystem integriert ist. Über den Verteiler 23 wird das Schlauchsystem auf die hier dargestellten vier Schläuche 33 bis 36 aufgeteilt. Der Schlauch 33 umfasst neben dem eigentlichen Schlauch 42 auch den Abstandshalter 43, wie dies in der Detaildarstellung der Fig. 4 zu erkennen ist.

Besonders vorteilhaft ist bei der vorliegenden Erfindung die Integration von drei - ggf. beliebig vielen - Domen in eine Domkassette und die gemeinsame Kopplung der Kanäle an die Sensoren über eine Andruckwalze und Klemmkante. Hierbei wirken die auf Andruckwalzen- und Klemmkantenseite in die Domkassette integrierten Stege als Federelemente, die den benötigten Anpressdruck der Membrane, die vorzugsweise auf den Bodenabschnitt der Druckdome angeordnet sind, auf die Sensoren herstellen.

Diese Konstruktion hat große Vorteile gegenüber der konventionellen Lösung mit Einzeldomen: Die Herstellung ist sehr viel günstiger und die Handhabung wird extrem vereinfacht. Statt wie bei den bisherigen Lösungen jeden Dom einzeln auf seinen Sensor zu stecken, genügt das Einlegen der Kassette und ein anschließender Knopfdruck mit dem die Anpresswalze über einen Aktuator in die "geschlossen" Position gedreht wird. Die vormals hohe Anzahl von benötigten, sterilen Einzelkomponenten ist insbesondere auf ein einziges Einmalprodukt reduziert.

Da in vielen Anwendungsgebieten die Sensoren oberhalb der Messorte im Körper platziert sein können, entstehen wegen der hydrostatischen Kraft der Wassersäule auch negative Drücke (niedriger als Atmosphärendruck) im Dom, die dann nicht durch Druck auf die Sensorfläche, sondern durch Zug gemessen werden. Zur Herstellung des dazu benötigten Saugeffektes müssen die Kontaktierungen zwischen Membranen und Sensoren komplett luftdicht ausgelegt sein. Dazu benötigt man einen Anpressdruck.

Figur 5 zeigt eine Explosionsansicht für die Zusammenstellung der Druckdomkassette 51 mit den Druckmesssensoren 54. Hierbei ist neben der Domkassette 51, die Andruckwalze 52, die Klemmkante 55 aufweisende Aufnahme 53 zu erkennen. Ferner sind mit dem Bezugszeichen 56 die Positionen im Bezug auf die Domkassette 51 dargestellt, die mit einem Druckmesswertgeber ausgestattet sind. In der Position 57 ist kein Druckmesswertgeber vorgesehen.

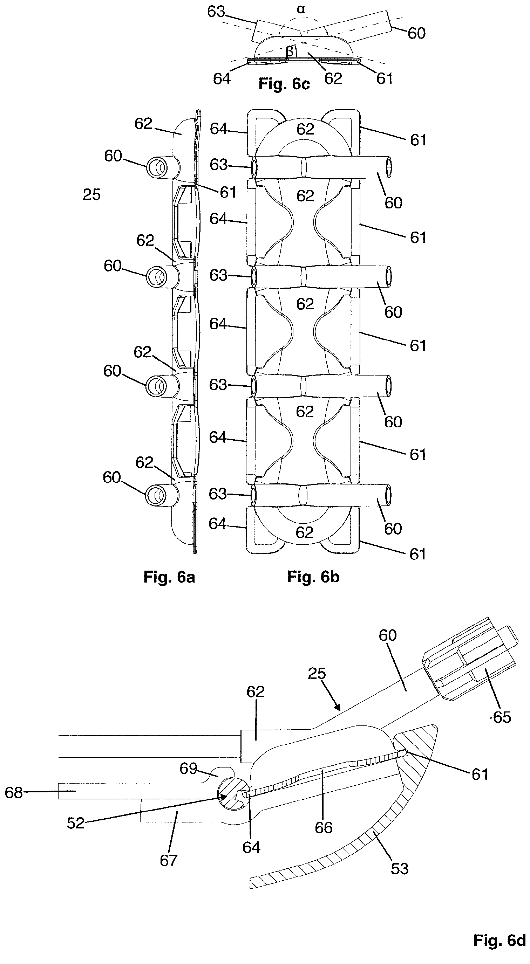

In den Figuren 6a bis 6d sind Detaildarstellungen der Domkassette 25 und deren Verriegelungsanordnung auf den Druckmesssensoren gezeigt. Dabei ist die Figur 6a eine Seitenansicht der Domkassette 25 dargestellt, bei welcher die Anschlüsse 60, der Andrucksteg 61 und Teile der Fluiddome 62 zu erkennen sind. In Figur 6b ist die Draufsicht der Domkassette 25 dargestellt, bei welcher zusätzlich die weiteren Anschlüsse 63 und den hinteren Andrucksteg 64 zu erkennen sind. Figur 6c ist eine weitere Seitenansicht, bei welcher neben den beiden Anschlüssen 60 und 63, auch die beiden Andruckstege 61 und 64 und die Winkelanordnung der Zuführungen 60 und 63 zueinander mit dem Winkel α und zum Boden mit dem Winkel β gezeigt sind.

Figur 6d zeigt die Anordnung der Domkassette 25 auf den Druckmesswertgebern, wobei die Domkassette 25 über den Andrucksteg 61 in der Aufnahme 53 ausgerichtet und mittels der Anpresswalze 52 und deren axialen Schlitzung in Kombination mit dem Verdrehen fixiert wird. Die Aufnahme 53 umfasst hierbei auch die Führung 69 und 68, als direkter oder indirekter Bestandteil der Aufnahme, die auch in den entsprechenden Positionen die Druckmesswertgeber (hier nicht dargestellt) umfasst. Neben der Aufnahme sind in dieser Darstellung auch die Anschlüsse 60 und 63 zu erkennen, wobei der Anschluss 60 auch einen Luer-Verschluss 65 aufweist.

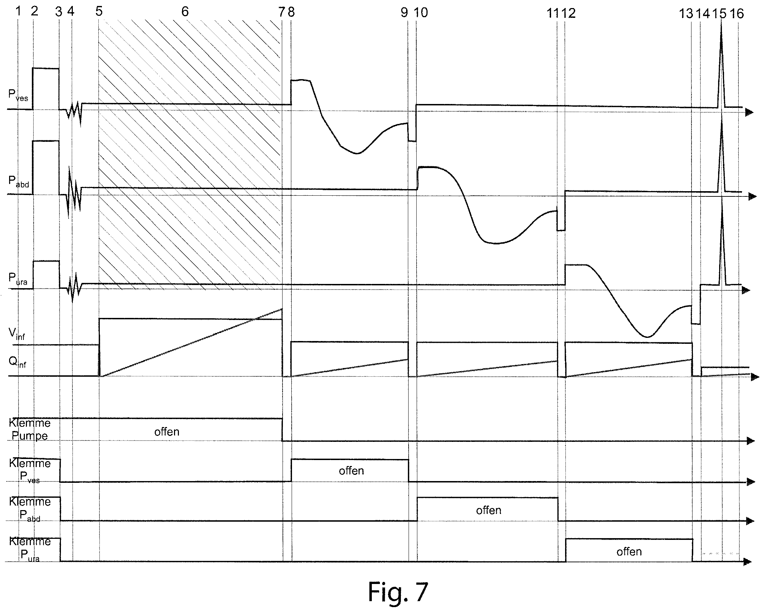

In Figur 7 ist über die Verfahrensschritte der Druckverlauf an den entsprechenden Positionen bzw. die Stellposition der Bauteile wiedergegeben. Dabei steht Pᵤᵣₐ, Pᵥₑₛ und P_{abd} für den Druck in den entsprechenden Druckdomen, V_{inf} und Qi_{nf} für das Infusionsvolumen und den Fluidstrom und Klemme-Pumpe, Klemme-Pᵤᵣₐ, Klemme-P_{abd} und Klemme-Pᵥₑₛ für die Schaltposition der entsprechenden Schlauchklemme. Das Verfahren wird nachfolgend beispielhaft beschrieben:
1) Der Benutzer legt die Domkassette 25 des Pumpenschlauchs 21 in das Gerät ein und führt den Einstichdorn 30 in den Infusionsbeutel 20 ein.
2) Der Verriegelungsmechanismus wird geschlossen, wodurch durch den Anpressdruck der Dome 25 auf die Sensoren ein Druckoffset entsteht.
3) Der Druckoffset wird automatisch nach einer Materialentspannungszeit von 5-10s ausgeglichen (zu Null gesetzt). Gleichzeitig werden die Schlauchklemmen 24 der Druckkanäle 26 und 27 geschlossen.
4) Der Benutzer platziert den transurethralen Katheter 29 in die Blase und den rektalen Katheter 28 in das Rektum des Patienten (falls nicht schon vorher geschehen) und verbindet den Pumpenschlauch 21 sowie die Druckübertragungsschläuche mit den Luer-Locks der Domkassette 25. Ab diesem Zeitpunkt kann der Benutzer die korrekte Platzierung der Katheter 28,29 anhand der am Gerät angezeigten Ruhedruckwerte, die jetzt über die Luftsäule der Druckübertragungsschläuche und Katheterlumen gemessen werden, überprüfen.
5) Wenn die Ruhedrücke plausible Werte zeigen, startet der Benutzer die vollautomatische Entlüftungs- und Nullstellungsprozedur per Tastendruck am Gerät, bei der zunächst die gesamte Infusionslinie entlüftet wird.
6) Im schraffiert dargestellten Zeitintervall messen alle Druckkanäle den Ruhedruck am jeweiligen Messort über die Luftsäule. Um zufällige Schwankungen durch Bewegung des Patienten oder Störungen zu minimieren wird der durchschnittlich gemessene Wert aller Kanäle während dieses Zeitraums berechnet und später als Ruhedruckwert zur Nullstellung verwendet.
7) Da das Volumen der Infusionslinie (Schlauch plus Fülllumen des Katheters) exakt bekannt ist und die beförderte Menge des Füllmediums durch den Volumentransducer bzw. Umdrehungen des Rollenrades der Pumpe 22 ständig gemessen wird, kann die Entlüftung vollautomatisch beendet werden, wenn die Wassersäule den Fülllumenausgang erreicht hat. Die Pumpenschlauchklemme 24 wird geschlossen.
8, 10, 12) Die Entlüftung der Druckübertragungsschläuche geschieht dann sequentiell immer nach dem gleichen Schema. Die jeweilige Schlauchklemme wird geöffnet, während alle anderen geschlossen sind und die Pumpe befördert das Füllmedium in definierter Füllgeschwindigkeit durch die jeweilige Druckübertragungslinie. Zu beachten ist hierbei, dass während des Befüllens wegen der sich aufbauenden Wassersäule zunehmend eine hydrostatische und zusätzlich eine dynamische Komponente hinzukommt, nicht mehr der tatsächliche Druck am Messort ermittelt wird..
9, 11, 13) Da die Volumina der Druckübertragungslinien bekannt sind und die Füllmenge ständig gemessen wird, kann der Entlüftungsvorgang vollautomatisch beendet werden, wenn die Wassersäule die Messlumenausgänge erreicht hat. Die Pumpe 22 wird gestoppt und die jeweilige Schlauchklemme 24 geschlossen. In diesem Moment misst das Gerät den momentanen Druck am Messort im Körper plus einen sich aus dem nicht bekannten Höhenunterschied zwischen Messort und Sensor ergebenden hydrostatischen Druck. Dieser wird jetzt vollautomatisch herausgerechnet, indem man softwaremäßig den jeweiligen Messkanal auf den in (6) über Luftsäule gemessenen Ruhedruck setzt.
14, 15) Die korrekte Platzierung der Katheter wird in einem letzten Schritt überprüft, indem man den Patienten Husten lässt und die dadurch entstehenden Druckspitzen vergleicht. Dazu werden die Schlauchklemmen 24 aller Kanäle, die Perfusion für die Messung benötigen (Pura in der Urodynamik), automatisch in die Perfusionstellung und die Pumpe 22 auf Perfusionsgeschwindigkeit gestellt.
16) Die Vorbereitungsphase ist abgeschlossen und die eigentliche Messung kann beginnen.

## Patentansprüche

1. Druckmesssystem,
mit wenigstens einem, mit einem Fluid befüllbaren, Messlumen (21, 26, 27, 28, 29),
wenigstens einem Schlauchsystem (23, 26, 27) zur Verbindung eines Fluidreservoirs (20) mit dem wenigstens einem Messlumen (21, 26, 27, 28, 29), und
einer Anordnung oder Vorrichtung (22) zur Beförderung des Fluids durch das wenigstens eine Schlauchsystem (23, 26, 27),
**dadurch gekennzeichnet, dass**
das Schlauchsystem (23, 26, 27) wenigstens eine erste Vorrichtung (25) zur Druckmessung in dem Fluid bestehend aus einem Kopplungselement (37), wenigstens einem Druckwandler (54) und wenigstens einer, mit dem Fluid befüllbaren Messkammer (62) aufweist, wobei das Kopplungselement (37) mit der Messkammer (62) über eine Membran (66) mechanisch mit der Messfläche des Druckwandlers (54) gekoppelt ist und die Messkammer (62) wenigstens zwei Anschlussstellen (60, 63) für einen Fluidstrom aufweist, und
das Schlauchsystem (23, 26, 27) zwischen der ersten Vorrichtung und der Zuführung des Fluids eine zweite Vorrichtung (24, 41) zur Regulation eines Fluidstroms mit einem fluiddurchströmbaren Schlauchabschnitt (42) und wenigstens einer Schlauchklemme (44) aufweist, und
die zweite Vorrichtung (24, 41) in die Regulationsstellungen offen, geschlossen und perfundiert einstellbar ist, wobei in der geschlossenen Stellung das Lumen des Schlauchabschnitts (42) vollständig geschlossen und in der perfundierten Stellung das Lumen des Schlauchabschnitts durch einen Abstandhalter (43) im Inneren des Schlauchabschnitts (42) teilweise, insbesondere geringfügig, offen ist.

2. Druckmesssystem nach Anspruch 1, **dadurch gekennzeichnet, dass** das Kopplungselement (37) eine Klemmkante (55) und wenigstens eine drehbar gelagerte, kraft- und/oder formschlüssig mit der Kopplungseinheit verbindbare Andruckwalze (52) aufweist und dass die wenigstens eine Messkammer (62) zwei gegenüberliegende äußere Stege (61, 64) aufweist, wobei einer der Stege (61, 64) in eine Klemmkante (55) und der andere Steg in die Andruckwalze (52) eingreift.

3. Druckmesssystem gemäß einem der Ansprüche 1 oder 2, **dadurch gekennzeichnet, dass**
die Schlauchklemme (41) ein Grundgehäuse mit einem Aufnahmebereich für den Schlauchabschnitt und ein, zwischen wenigstens zwei, bevorzugt in drei Regulationsstellungen bewegbares, Klemmelement (44) aufweist und das Klemmelement (44) in Wechselwirkung mit Abschnitten des Aufnahmebereichs als Andruckstellen in wenigstens einer Regulationsstellung auf den Schlauchabschnitt und dessen Lumen einwirkt und dass im Bereich wenigstens einer Regulationsstellung im Lumen des Schlauchabschnitts (42) ein Abstandshalter (43) angeordnet ist.

4. Druckmesssystem gemäß Anspruch 3, **dadurch gekennzeichnet, dass**
das Klemmelement (44) drehbeweglich angeordnet ist und sich radial um den Drehpunkt erstreckt und insbesondere der zum Drehpunkt nächstgelegenen Abschnitt des Klemmelements mit wenigstens einer Andruckstelle zusammenwirkt.

5. Druckmesssystem gemäß einem der vorstehenden Ansprüche, **dadurch gekennzeichnet, dass**
der Schlauchabschnitt (42) ein Lumen, d.h. einen lichten Innendurchmesser, aufweist, welcher zwischen 0,2 mm und 10 mm, vorzugsweise zwischen 0,5 mm und 5 mm und insbesondere bei ca. 3 mm, liegt.

6. Druckmesssystem gemäß einem der vorstehenden Ansprüche, **dadurch gekennzeichnet, dass**
der Abstandhalter (43) integraler Bestandteil eines Schlauchabschnitts (42) ist und insbesondere in diesem Abschnitt das Lumen des Schlauchs (21) ungleichmäßig reduziert und/oder der Abstandhalter (43) durch ein längliches, insbesondere ein faden- oder stabförmiges, das Lumen des Schlauchs ungleichmäßig reduzierendes Element, gebildet wird, welches wenigstens abschnittsweise mit dem Schlauchabschnitt (42) kraft-, form- und/oder stoffschlüssig verbunden ist.

7. Druckmesssystem gemäß einem der Ansprüche 1 bis 6, **dadurch gekennzeichnet, dass**
die Reduzierung des Lumens durch den Abstandhalter (43) zwischen 0,01 % bis 5 %, bevorzugt zwischen 0,1 % und 1 % und insbesondere bei ca. 0,5 %, liegt und dadurch das Lumen des Schlauchabschnittes (42) in der perfundierten Stellung derart geringfügig offen gehalten wird, dass ein Volumenstrom des Fluids zwischen 0,1 ml/min und 8 ml/min, vorzugsweise zwischen 0,5 ml/min und 5 ml/min, bereitgestellt wird.

8. Druckmesssystem nach einem der Ansprüche 1 bis 7, **dadurch gekennzeichnet, dass**
der Innenraum der Messkammer (62) eine im Wesentlichen kreisrunde Grundfläche aufweist.

9. Druckmesssystem nach Anspruch 8, **dadurch gekennzeichnet, dass**
die Grundfläche offen ist und/oder die Membran (66) die offene Grundfläche abdeckt und/oder die Membran (66) aus einem Material hergestellt ist, welches aus einer Gruppe ausgewählt wird, welche Silikon, Latex, Kautschuk, Kombinationen hiervon und dergleichen enthält.

10. Druckmesssystem nach einem der vorstehenden Ansprüche, **dadurch gekennzeichnet, dass**
der Druckwandler (54) ein mechano-elektrischer Druckwandler ist und vorzugsweise wenigstens der Messbereich des Druckwandlers formschlüssig von der Membran (66) bedeckt wird und/oder der Messbereich des Druckwandlers (54) zentrisch in Bezug auf die Grundfläche der Messkammer (62) angeordnet ist.

11. Druckmesssystem gemäß einem der vorstehenden Ansprüche, **dadurch gekennzeichnet, dass**
wenigstens die Schlauchklemme (41) und/oder das Kopplungselement (37) und/oder die Messkammer (62) wenigstens abschnittsweise aus einem Material hergestellt wird, das aus einer Gruppe ausgewählt ist, welche duro- und thermoplastische Kunststoffe und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan ungesättigtes Polyesterharz, Acrylnitril-Butadien-Styrol, Acrylester-Styrol-Acrylnitril, Metalle wie zum Beispiel Edelstahl, Aluminium, Kombinationen hiervon und dergleichen umfasst.

12. Verfahren zur automatischen Entlüftung und Nullstellung des Druckmesssystems gemäß einem der vorstehenden Ansprüche, nach Positionierung des wenigstens einen Messlumens (21, 26, 27, 28, 29), mit den Schritten:
• Messung des Ruhedrucks am Druckwandler bei luftgefüllten Schlauchsystem (23, 26, 27) und Messlumen (21, 26, 27, 28, 29) und geschlossenen Schlauchklemmen der Druckübertragungslinien;
• Befüllen des Schlauchsystems (23, 26, 27) und des Messlumens (21, 26, 27, 28, 29) mit Fluid aus einem Fluidreservoir (20) und Schließen einer Pumpenschlauchklemme (24);
• Sequentielle Entlüftung einer Druckübertragungslinie durch:
• Öffnen der jeweiligen Schlauchklemme der Druckübertragungslinie während alle anderen geschlossen sind, wobei eine Pumpe (22) das Füllmedium in definierter Füllgeschwindigkeit durch die Druckübertragungslinie fördert;
• Schließen der jeweiligen Schlauchklemme der Druckübertragungslinie (24);
• Korrektur des durch den Höhenunterschied zwischen dem Druckwandler (54) bzw. der Messkammer (62) und dem Messlumen (21, 26, 27, 28, 29) bzw. dem Messort wirkenden hydrostatischen Druck durch Abgleich mit dem vorher bestimmten Ruhedruck.

13. Verfahren gemäß Anspruch 12, **dadurch gekennzeichnet, dass**
der gemessene Ruhedruck an ein Steuergerät (31) übertragen wird und aus mehreren Messwerten ein mittlerer Ruhedruck bestimmt wird.

14. Verfahren gemäß Anspruch 13, **dadurch gekennzeichnet, dass**
die Korrektur des durch den Höhenunterschied zwischen der Messkammer (62) und dem Druckwandler (54) wirkenden hydrostatischen Drucks durch Abgleich mit dem mittleren Ruhedruck erfolgt.

15. Verwendungeines Druckmesssystems nach einem derAnsprüche 1 bis 11 im Bereich der Urodynamik und Gastroenterologie, insbesondere zur Blasen-, Rektal- und Urethraldruckmessung.

## Claims

1. A pressure-measuring system
having at least one measuring lumen (21,26, 27, 28, 29) which can be filled with a fluid,
at least one tube system (23, 26, 27) for connecting a fluid reservoir (20) to the at least one measuring lumen (21, 26, 27, 28,29), and
an assembly or device (22) for conveying the fluid through the at least one tube system (23, 26, 27),
**characterised in that**
the tube system (23, 26, 27) includes at least one first device (25) for measuring pressure in the fluid, comprising a coupling element (37), at least one pressure transducer (54) and at least one measuring chamber (62) which can be filled with the fluid, wherein the coupling element (37) with the measuring chamber (62) is mechanically coupled via a membrane (66) to the measuring area of the pressure transducer (54) and the measuring chamber (62) has at least two connection points (60, 63) for a fluid flow, and
between the first device and the supply of the fluid, the tube system (23, 26, 27) includes a second device (24, 41) for regulating a fluid flow with a tube section (42) through which fluid can flow and at least one tube clamp (44), and
the second device (24, 41) can be set to the regulation positions open, closed and perfused, wherein in the closed position the lumen of the tube section (42) is completely closed and in the perfused position, through a spacer (43) inside the tube section (42), the lumen of the tube section is partially, more particularly slightly, open.

2. The pressure-measuring system of claim 1, **characterised in that**
the coupling element (37) has a clamping edge (55) and at least one rotatably borne pressing roller (52) that can be connected in a frictional and/or form-fitting manner to the coupling device and that the at least one measuring chamber (62) has two webs (61, 64) arranged opposite each other, wherein one of the webs (61, 64) is engaged with a clamping edge (55) and the other web with the pressing roller (52).

3. The pressure-measuring system according to one of claims 1 or 2, **characterised in that**
the tube clamp (41) has a basic housing with an accommodating area for the tube section and a clamping element (44) that can be moved between at least two, and preferably in three, regulation positions and the clamping element (44) in interaction with sections of the accommodating area as pressing points in at least one regulation position acts on the tube section and its lumen and that in the area of at least one regulation position a spacer (43) is arranged in the lumen of the tube section (42).

4. The pressure-measuring system according to claim 3, **characterised in that**
the clamping element (44) is arranged in a rotating manner and extends radially around the centre of rotation and, more particularly, the section of the clamping element closest to the centre of rotation interacts with at least one pressing point.

5. The pressure-measuring system according to any preceding claim, **characterised in that**
the tube section (42) has a lumen, i.e. a clear inside diameter, which is between 0.2mm and 10mm, preferably between 0.5mm and 5mm, and more particularly about 3mm.

6. The pressure-measuring system according to any preceding claim, **characterised in that**
the spacer (43) is an integral component of a tube section (42) and in particular in this section irregularly reduces the lumen of the tube (21), and/or the spacer (43) is formed by an elongate, more particularly thread-like or rod-like element that irregularly reduces the lumen of the tube and is connected in at least a portion thereof to the tube section (42) in a frictional, form-fitting or adhesive manner.

7. The pressure-measuring system according to any of claims 1 to 6, **characterised in that**
the reduction of the lumen by the spacer (43) is between 0.01% and 5%, preferably between 0.1% and 1%, and more particularly about 0.5%, and thereby in the perfused position the lumen of the tube section (42) is kept slightly open so that a volumetric flow of the fluid of between 0.1ml/min and 8ml/min, preferably between 0.5ml/min and 5ml/min, is provided.

8. The pressure-measuring system of any of claims 1 to 7, **characterised in that**
the interior of the measuring chamber (62) has an essentially circular base area.

9. The pressure-measuring system of claim 8, **characterised in that**
the base area is open and/or the membrane (66) covers the open base area and/or the membrane (66) is made of a material selected from a group that contains silicone, latex, rubber, combinations thereof, and the like.

10. The pressure-measuring system of any preceding claim, **characterised in that**
the pressure transducer (54) is an electromechanical pressure transducer and preferably at least the measuring portion of the pressure transducer is covered by the membrane (66) in a form-fitting manner, and/or is arranged centrically in relation to the base area of the measuring chamber (62).

11. The pressure-measuring system according to any preceding claim, **characterised in that**
at least the tube clamp (41) and/or the coupling element (37) and/or the measuring chamber (62) are made, in at least a portion thereof, of a material selected from a group comprising duroplastics and thermoplastics, and more particularly polyphenylene sulfide, polypropylene, poly(1-butene), polyvinyl chloride, polyvinylidene chloride, polymethyl metaacrylate, polyacrylonitrile, polystyrene, polyacetal, polyvinyl alcohol, polyvinyl acetate, ionomers, fluoroplastic, polyethylene, polyamide, and particularly a partially aromatic polyamide, polycarbonate, polyester, polyphenylene oxide, polysulfone, polyvinyl acetal, polyurethane, and chlorinated polyether, cellulose nitrate, cellulose acetate, cellulose ether, phenolic resin, urea resin, thiourea resin, melamine resin, alkyl resin, allyl resin, silicone, polyimide, polybenzimidazole, epoxy resin, casein plastic, cross-linked polyurethane, unsaturated polyester resin, acrylonitrile butadiene styrene, acrylester styrene acrylonitrile, metals such as stainless steel, aluminum, combinations thereof, and the like.

12. A method for automatic de-aeration and zeroing of the pressure-measuring system according to any preceding claim after positioning the at least one measuring lumen (21, 26, 27, 28, 29), the method comprising the steps of:
• measuring the rest pressure on the pressure transducer with the tube system (23, 26, 27) and measuring lumen (21, 26, 27, 28, 29) filled with air and the tube clamps of the pressure transfer lines closed;
• filling the tube system (23, 26, 27) and the measuring lumen (21, 26, 27, 28, 29) with fluid from a fluid reservoir (20) and closing a pump tube clamp (24);
• sequential de-aeration of a pressure transfer line by:
• opening the respective tube clamp of the pressure transfer line while all the others are closed, with a pump (22) conveying the filled medium through the pressure transfer line at a defined filling rate;
• closing the respective tube clamp of the pressure transfer line (24);
• correction of the hydrostatic pressure exerted by the height difference between the pressure transducer (54) or the measuring chamber (62) and the measuring lumen (21, 26, 27, 28, 29) or the measuring location by comparison with the previously determined rest pressure.

13. The method according to claim 12, **characterised in that**
the measured rest pressure is transmitted to a control device (31) and from several measurements a mean rest pressure is determined.

14. The method according to claim 13, **characterised in that**
the correction of the hydrostatic pressure exerted by the height difference between the measuring chamber (62) and the pressure transducer (54) takes place through comparison with the mean rest pressure.

15. Use of a pressure measuring system according to any of claims 1 to 11 in the field of urodynamics and gastroenterology, and more particularly for measuring bladder pressure, rectal pressure, and urethral pressure.

## Revendications

1. Système de mesure de la pression
avec au moins un lumen de mesure, remplissable par un fluide (21, 26, 27, 28, 29),
au moins un système de tuyaux (23, 26, 27) en vue de la liaison d'un réservoir à fluide (20) avec au moins l'un des lumens de mesure (21, 26, 27, 28, 29), et
une disposition ou un dispositif (22) en vue du transport du fluide à travers au moins l'un des systèmes de tuyaux (23, 26, 27),
**caractérisé en ce que**
le système de tuyaux (23, 26, 27) présente au moins un premier dispositif (25) en vue de la mesure de la pression dans le fluide, se composant d'un élément de couplage (37), au moins un convertisseur de pression (54) et au moins une chambre de mesure remplissable par le fluide (62), l'élément de couplage (37) étant accouplé avec la chambre de mesure (62) via une membrane (66) de manière mécanique avec la surface de mesure du convertisseur de pression (54) et la chambre de mesure (62) présentant au moins deux points de jonction (60, 63) pour un courant de fluide, et
le système de tuyaux (23, 26, 27) présente, entre le premier dispositif et l'alimentation du fluide, un second dispositif (24, 41) en vue de la régulation d'un courant de fluide avec une section de tuyaux traversable par du fluide (42) et au moins un collier de serrage (44), et
le second dispositif (24, 41) est réglable dans les positions de régulation ouverte, fermée et perfusée, sachant que dans la position fermée, le lumen de la section de tuyau (42) est complètement fermé et que dans la position perfusée, le lumen de la section de tuyau est ouvert partiellement, notamment légèrement par un écarteur (43) à l'intérieur de la section de tuyau (42).

2. Système de mesure de la pression selon la revendication 1, **caractérisé en ce que**
l'élément de couplage (37) présente une arête de serrage (55) et au moins un cylindre de compression (52) monté sur paliers tournants, reliable par adhérence et/ou mécaniquement avec l'unité de couplage et **en ce que** au moins l'une des chambres de mesure (62) présente deux entretoises extérieures opposées (61, 64), une des entretoises (61, 64) s'engrenant dans une arête de serrage (55) et l'autre entretoise s'engrenant dans le cylindre de compression (52).

3. Système de mesure de la pression selon une quelconque des revendications 1 ou 2, **caractérisé en ce que**
le collier de serrage (41) présente un corps de base avec un secteur de réception pour la section de tuyau et un élément de serrage (44) mobile entre au moins deux, de préférence dans trois positions de régulation et l'élément de serrage (44) agit en interaction avec des sections du secteur de réception comme points d'appui dans au moins une position de régulation sur la section de tuyau et son lumen et **en ce que**, dans le secteur d'au moins une position de régulation dans le lumen de la section de tuyau (42), un écarteur (43) est disposé.

4. Système de mesure de la pression selon la revendication 3, **caractérisé en ce que**
l'élément de serrage (44) est disposé de manière mobile rotative et s'étend radialement autour du point de rotation et notamment **en ce que** la section suivante de l'élément de serrage proche du point de rotation, interagit avec au moins un point d'appui.

5. Système de mesure de la pression selon une quelconque des revendications précédentes,
**caractérisé en ce que**
la section de tuyau (42) présente un lumen, c'est-à-dire un diamètre interne écarté, qui se trouve entre 0,2 mm et 10 mm, de préférence entre 0,5 mm et 5 mm et notamment à env. 3 mm.

6. Système de mesure de la pression selon une quelconque des revendications précédentes,
**caractérisé en ce que**
l'écarteur (43) forme partie intégrante d'une section de tuyau (42) et notamment dans cette section, réduit le lumen du tuyau (21) de manière irrégulière et/ou l'écarteur (43) est constitué par un élément oblong, notamment filiforme ou en forme de barre, réduisant de manière irrégulière le lumen du tuyau, qui est relié au moins par section avec la section de tuyau (42) par adhérence, mécaniquement et/ou par composition de matière.

7. Système de mesure de la pression selon une quelconque des revendications 1 à 6, **caractérisé en ce que**
la réduction du lumen par l'écarteur (43) se trouve entre 0,01 % à 5 %, de préférence entre 0,1 % et 1 % et notamment à env. 0,5 %, et le lumen de la section de tuyau (42) est ainsi maintenu ouvert de manière tellement légère dans la position perfusée, qu'un débit volumique du fluide est mis à disposition entre 0,1 ml/min et 8 ml/min, de préférence entre 0,5 ml/min et 5 ml/min.

8. Système de mesure de la pression selon une quelconque des revendications 1 à 7, **caractérisé en ce que**
l'intérieur de la chambre de mesure (62) présente une surface de base circulaire pour l'essentiel.

9. Système de mesure de la pression selon la revendication 8, **caractérisé en ce que**
la surface de base est ouverte et/ou la membrane (66) couvre la surface de base ouverte et/ou la membrane (66) est fabriquée à partir d'une matière qui est sélectionnée à partir d'un groupe qui contient de la silicone, du latex, du caoutchouc, des combinaisons de ceux-ci et similaires.

10. Système de mesure de la pression selon une quelconque des revendications précédentes,
**caractérisé en ce que**
le convertisseur de pression (54) est un convertisseur de pression mécano-électrique et de préférence au moins le secteur de mesure du convertisseur de pression est couvert mécaniquement par la membrane (66) et/ou le secteur de mesure du convertisseur de pression (54) est disposé de manière centrée par rapport à la surface de base de la chambre de mesure (62).

11. Système de mesure de la pression selon une quelconque des revendications précédentes,
**caractérisé en ce que**
au moins le collier de serrage (41) et/ou l'élément de couplage (37) et/ou la chambre de mesure (62) est fabriqué au moins par section en une matière qui est sélectionnée à partir d'un groupe qui comporte de préférence des duroplastiques et des thermoplastiques et notamment du polysulfure de phénylène, du polypropylène, du poly-1-butylène, du chlorure de polyvinyle, du chlorure de polyvinylidène, du polyméthacrylate de méthyle, du polyacrylonitrile, du polystyrène, du polyacétal, de l'alcool polyvinylique, du polyacétate de vinyle, des ionomères, du plastique fluoré, du polyéthylène, du polyamide, notamment un polyamide aromatique partiel, du polycarbonate, du polyester, du polyphénylène-oxyde, du polysulfone, de l'acétal polyvinylique, du polyuréthane, et du polyéther chloré, du nitrate de cellulose, de l'acétate de cellulose, de l'éther de cellulose, de la résine phénolique, de la résine d'urée, de la résine thio-urée, de la résine de mélamine, de la résine d'alcoyle, de la résine allylique, de la silicone, du polyimide, du polybenzimidazole, de la résine époxy, du plastique à la caséine, du polyuréthane réticulé, de la résine polyester non saturée, de l'acrylonitrile-butadiène-styrène, de l'ester acrylique-styrène-acrylonitrile, des métaux comme par exemple l'inox, l'aluminium, des combinaisons de ceux-ci et des produits similaires.

12. Procédé en vue de la purge automatique et de la remise à zéro du système de mesure de la pression selon une quelconque des revendications précédentes, après le positionnement d'au moins l'un des lumens de mesure (21, 26, 27, 28, 29), avec les étapes de :
• mesure de la pression de repos sur le convertisseur de pression en présence d'un système de tuyaux (23, 26, 27) et d'un lumen de mesure (21, 26, 27, 28, 29) remplis d'air ; et sur les colliers de serrage des lignes de transmission de la pression ;
• remplissage du système de tuyaux (23, 26, 27) et du lumen de mesure (21, 26, 27, 28, 29) par du fluide issu d'un réservoir à fluide (20) et fermeture d'un collier de serrage de pompe (24) ;
• purge séquentielle d'une ligne de transmission de la pression par :
• ouverture du collier de serrage respectif de la ligne de transmission de la pression pendant que tous les autres sont fermés, une pompe (22) transportant le fluide de remplissage à une vitesse de remplissage définie à travers la ligne de transmission de la pression ;
• fermeture du collier de serrage respectif de la ligne de transmission de la pression (24) ;
• correction de la pression hydrostatique agissant par la différence de hauteur entre le convertisseur de pression (54) et/ou la chambre de mesure (62) et le lumen de mesure (21, 26, 27, 28, 29) et/ou le lieu de mesure par alignement avec la pression de repos déterminée auparavant.

13. Procédé selon la revendication 12, **caractérisé en ce que**
la pression de repos mesurée est transmise à un appareil de commande (31) et à partir de plusieurs valeurs de mesure, une pression de repos moyenne est déterminée.

14. Procédé selon la revendication 13, **caractérisé en ce que**
la correction de la pression hydrostatique agissant par la différence de hauteur entre la chambre de mesure (62) et le convertisseur de pression (54) s'effectue par alignement avec la pression de repos moyenne.

15. Utilisation d'un système de de mesure de la pression selon une quelconque des revendications 1 à 14 dans le secteur de l'urodynamique et de la gastroentérologie, notamment pour la mesure des pressions vésicale, rectale et urétrale.
